# EUROPÄISCHE PATENTSCHRIFT

(11) **EP 3 579 894 B1**
(45) Veröffentlichungstag und Bekanntmachung des Hinweises auf die Patenterteilung: **09.12.2020**
(21) Anmeldenummer: 18704229.6
(22) Anmeldetag: 08.02.2018
(51) Int. Cl.: A61M 1/10, A61M 1/12

(54) **KATHETERPUMPE MIT ANTRIEBSEINHEIT UND KATHETER**
CATHETER PUMP WITH DRIVE UNIT AND CATHETER
POMPE A CATHETER AVEC UNITE D'ENTRAINEMENT ET CATHETER

(30) Priorität: 13.02.2017 DE 102017102825
(43) Veröffentlichungstag der Anmeldung: 18.12.2019
(73) Patentinhaber: Cardiobridge GmbH, 72379 Hechingen (DE)
(72) Erfinder: EPPLE, Klaus, 72414 Rangendingen (DE); FRITZ, Andreas, 72116 Moessingen (DE)
(74) Vertreter: DREISS Patentanwälte PartG mbB
(86) Internationale Anmeldenummer: PCT/EP2018/053130
(87) Internationale Veröffentlichungsnummer: WO 2018/146176

(56) Entgegenhaltungen:
- WO-A1-2010/127871
- WO-A2-2009/046790
- WO-A2-2016/118781

## Beschreibung

Die Erfindung betrifft eine Katheterpumpe mit einer Antriebseinheit und einem Katheter, wobei der Katheter einen Pumpenkopf zum Einführen in das arterielle Gefäßsystem wie zum Beispiel die Aorta oder das Herz, eine verdrehbar angeordnete Rotorwelle zum Antrieben eines am Pumpenkopf vorgesehenen, expandierbaren Förderelements und an seinem freien Ende einen mit der Rotorwelle drehfest verbundenen, um eine Koppelachse rotierbaren Koppelabschnitt aufweist, und wobei die Antriebseinheit einen Antrieb und einen vom Antrieb um eine Antriebsachse rotierenden antreibbaren Antriebsabschnitt aufweist.

Derartige Katheterpumpen sind beispielsweise aus der EP 2 288 392 B1 bekannt und dort in Figur 1a gezeigt. Als rotierendes Förderelement kann beispielsweise, wie in der EP 2 288 392 B1 beschrieben, ein Rotor mit ausklappbaren Propellern Verwendung finden, der am distalen Ende des Katheters vorgesehen ist. Ebenso ist denkbar, dass anders ausgebildete Förderelemente, wie beispielsweise helixartig ausgebildete Wendel Verwendung finden können.

Eine Katheterpumpe mit Merkmalen des Oberbegriffs des Patentanspruchs 1 ist aus der WO 2010/127871 A1 bekannt. Weitere, ähnliche Pumpen offenbaren die WO 2016/118781 A1 und WO 2009/046790 A2. Eine Blutpumpe mit einer Magnetkopplung ist aus der DE 10 2006 036 948 A1 bekannt.

Katheterpumpen werden als temporäres Kreislaufunterstützungssystem in die Aorta von Patienten eingesetzt, insbesondere dann, wenn das natürliche Herz nicht in der Lage ist, den Körper mit ausreichend Sauerstoff versetztem Blut zu versorgen. Das Förderelement und die Rotorwelle werden dabei mit vergleichsweise hohen Drehzahlen im Bereich von 7000 bis 15000 Umdrehungen pro Minute und insbesondere im Bereich von 10000 bis 13000 Umdrehungen betrieben. Dabei hat sich eine Geräuschentwicklung, insbesondere im Bereich der Kopplung des Koppelabschnitts mit dem Antriebsabschnitt als problematisch herausgestellt. Gerade bei diesen hohen Drehzahlen treten störende Vibrations- und Pfeifgeräusche auf. Der Pumpenkopf der Katheterpumpe kann insbesondere nach einer Operation mehrere Tage in der Aorta verbleiben.

Der vorliegenden Erfindung liegt deshalb die Aufgabe zugrunde, eine eingangs beschriebene Katheterpumpe bereitzustellen, die eine betriebssichere Kopplung des Koppelabschnitts mit dem Antriebsabschnitt aufweist und dennoch geräuscharm betrieben werden kann.

Diese Aufgabe wird mit einer Katheterpumpe mit den Merkmalen des Anspruchs 1 gelöst. Dadurch kann erreicht werden, dass im Nenndrehzahlbereich, also insbesondere bei einer Drehzahl von 8000 bis 13000 Umdrehungen pro Minute, der Koppelabschnitt aufgrund der Magnete in eine definierte Richtung gedrängt wird und eine stabile Lage einnimmt. Dies führt dazu, dass zum einen eine sichere Magnetkopplung gewährleistet wird und zum anderen ein sehr geräuscharmes Betreiben der Katheterpumpe ermöglicht wird. Dabei hat sich herausgestellt, dass dann, wenn die Koppelachse keinen Versatz zur Antriebsachse aufweist, aufgrund des sich drehenden Magnetfelds der Magnetelemente der Koppelabschnitt während der Rotation keine definierte Radiallage einnimmt und zwischen verschiedenen, undefinierten Positionen während der Rotation wechselt, was insgesamt zu störenden Vibrationen und Geräuschen führt. Dabei ist vorgesehen, dass der Abstand a im Bereich von 0,7 mm bis 3,5 mm und vorzugsweise im Bereich von 1 mm 3 mm und weiter vorzugsweise im Bereich von 2 mm liegt. Dieser vergleichsweise geringe Achsabstand reicht aus, um ein geräuscharmes Betreiben der Katheterpumpe zu gewährleisten.

Ferner ist vorteilhaft, wenn der Koppelabschnitt zum Antriebsabschnitt in axialer Richtung einen Abstand m aufweist, wobei der Abstand m im Bereich von 1 mm bis 6 mm, vorzugsweise im Bereich von 3 mm bis 5 mm und weiter vorzugsweise im Bereich von 3,5 mm liegt. Durch einen derartigen Abstand m kann insbesondere eine fluiddichte Anordnung des Koppelabschnitts zum Antriebsabschnitt bereitgestellt werden, wobei dennoch ausreichend Drehmomente übertragen werden können.

Ferner hat sich als vorteilhaft herausgestellt, wenn die Rotorwelle einen Durchmesser d aufweist, wobei der Abstand a im Bereich des 0,25- bis 1,5-fachen und vorzugsweise im Bereich des 0,5 - bis 1,2-fachen des Durchmessers d liegt. Auch diese Kombination hat zu einem vergleichsweise geräuscharmen Arbeiten der Katheterpumpe geführt.

Der Durchmesser d kann dabei vorzugsweise im Bereich von 0,7 mm bis 4,5 mm und vorzugsweise im Bereich von 1,5 mm bis 3 mm und weiter vorzugsweise im Bereich von 2 mm liegen. Auch dies führt zu einem geräuscharmen Betrieb.

Um eine sichere Kopplung des Koppelabschnitts mit dem Antriebsabschnitt zu gewährleisten, ist vorteilhaft, wenn die Antriebseinheit eine Aufnahme für den Koppelabschnitt aufweist, wobei der Koppelabschnitt in der Aufnahme eine bezüglich des Antriebsabschnitts in Querrichtung um den Abstand a versetzte Betriebslage einnimmt. Durch Vorsehen der Aufnahme kann folglich ein definiertes Anordnen des Koppelabschnitts bezüglich des Antriebsabschnitts gewährleistet werden.

Gemäß der Erfindung kann zudem vorgesehen sein, dass die Aufnahme oder eine die Aufnahme begrenzende Wandung ein oder mehrere Dämpfungselemente vorsieht, die zur Schwingungsdämpfung gegen den Koppelabschnitt wirken. Dadurch können ungewollte Restschwingungen des Koppelabschnitts sicher vermieden werden.

Der Koppelabschnitt kann dabei einen an dem freien Ende der Rotorwelle angeordneten Magnethalter mit daran angeordneten Magnetelementen aufweisen. Die Magnetelemente können dabei ringartig um die Koppelachse verlaufend angeordnet sein. Neben den Magnetelementen können auch Metallelemente oder Metallringe zur definierten Ausbreitung des Magnetfelds Verwendung finden.

Ferner ist vorteilhaft, wenn der Koppelabschnitt von einer fluiddichten Kappe umgeben ist. Die Kappe kann dabei in Verlängerung eines die Rotorwelle umgebenden Außenkatheters oder einer entsprechenden Hülse angeordnet sein, so dass die Pumpenwelle insgesamt fluiddicht im Katheter und auch der Koppelabschnitt fluiddicht in der Kappe angeordnet ist.

Gemäß der Erfindung ist weiterhin denkbar, dass die Kappe an der dem Antriebsabschnitt zugewandten Seite und/oder die Aufnahme an der dem Koppelabschnitt zugewandten Seite Zentrierschrägen zum Zentrieren der Kappe und damit des Koppelabschnitts in der Betriebslage aufweist. Hierdurch kann gewährleistet werden, dass die Kappe und damit der Koppelabschnitt um den Abstand a versetzt zur Antriebsachse in die Aufnahme einsetzbar ist.

Der Katheter kann einen Außenkatheter und einen Innenkatheter vorsehen, wobei im Innenkatheter die Rotorwelle drehbar angeordnet ist. Ferner kann Schmier- und Spülflüssigkeit vorgesehen sein, die im Betrieb dem Pumpenkopf zugeführt wird, um dort vorgesehene Lagerstellen zu schmieren und zu spülen. Die Schmier- und Spülflüssigkeit kann dabei insbesondere über ein zwischen dem Außenkatheter und dem Innenkatheter vorgesehenes Lumen hin zum Pumpenkopf geführt. Die rückgeführte Schmier- und Spülflüssigkeit kann über ein zwischen der Rotorwelle und dem Innenkatheter vorgesehenes Lumen rückgeführt werden. Dadurch kann eine Vermischung der Spülflüssigkeit, die hin zum Pumpenkopf transportiert wird, und der Spülflüssigkeit, die rückgeführt wird, vermieden werden.

Die Kappe kann dabei einen Auslauf aufweisen und der Katheter kann dabei so ausgebildet sein, dass die Schmier-und Spülflüssigkeit, die die Rotorwelle im Betrieb umspült, durch die Kappe und über den Auslauf aus der Kappe, und damit aus dem Katheter, abgeführt wird. Ein derartiges Spülen der Rotorwelle im Katheter dient insbesondere zum Schmieren und Abtransportieren des Abriebs und damit zu einem leichtgängigen Rotieren der Rotorwelle im Katheter.

Um zu verhindern, dass Verunreinigungen den Antriebsabschnitt erreichen können, ist vorteilhaft, wenn die Antriebseinheit zwischen der Aufnahme und dem Antriebsabschnitt einen fluiddichten Wandungsabschnitt aufweist. Gemäß dieser Ausführungsform können dann zwischen dem Koppelabschnitt und dem Antriebsabschnitt zum einen der Wandungsabschnitt und zum anderen die Kappe vorgesehen sein. Aufgrund des dennoch geringen Abstands zwischen Antriebsabschnitt und Koppelabschnitt und der geeigneten Auswahl der Magnete kann dennoch ein sicheres Drehkoppeln des Antriebsabschnitts mit dem Koppelabschnitt gewährleistet werden.

Ferner kann vorgesehen sein, dass die Magnetelemente am Koppelabschnitt und/oder am Antriebsabschnitt zur Drehkoppelung so ausgelegt sind, dass bei einem Grenzdrehmoment die Magnetkoppelung abreist. Hierdurch kann ein Überlastschutz bereitgestellt werden.

Weitere Ausführungsformen und vorteilhafte Ausgestaltungen der Erfindung sind der nachfolgenden Beschreibung zu entnehmen, anhand derer ein Ausführungsbeispiel der Erfindung näher beschrieben und erläutert wird.

Es zeigen:
Figur 1 eine Katheterpumpe mit einer Antriebseinheit und einem Katheter;
Figur 2 eine Antriebseinheit einer erfindungsgemäßen Katheterpumpe in Betriebslage und
Figur 3 einen vergrößerten Ausschnitt eines Längsschnitts durch den Koppelabschnitt und Antriebsabschnitt der Antriebseinheit gemäß Figur 2.

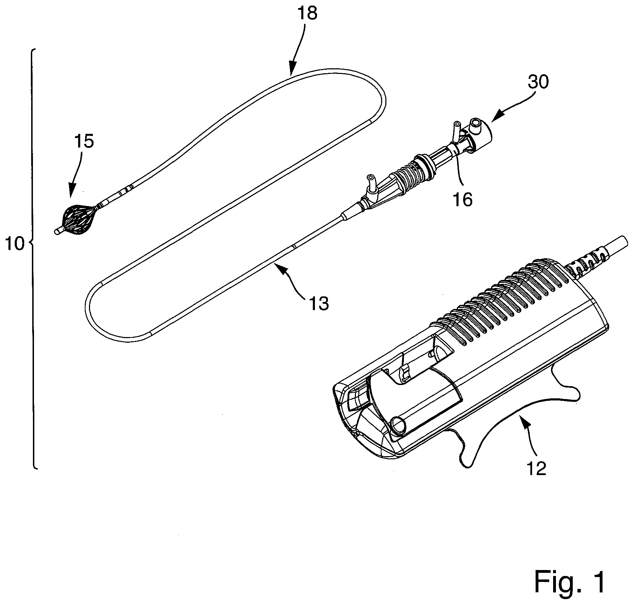

In der Figur 1 ist eine Katheterpumpe 10 mit einer Antriebseinheit 12 und ein mit der Antriebseinheit 12 koppelbarer Katheter 18 gezeigt. Der Katheter 18 weist an seinem distalen Ende einen Pumpenkopf 15 zum Einführen in das arterielle Gefäßsystem wie zum Beispiel die Aorta oder das Herz auf. Im Katheter 18 ist eine Rotorwelle 32 vorgesehen, mittels der ein im Pumpenkopf 15 vorgesehenes Förderelement, beispielsweise einen Rotor mit ausklappbaren Propellern, in Rotation versetzbar ist. An seinem proximalen Ende 16 sieht der Katheter 18 einen Kopplungsabschnitt 30 vor, der in die Antriebseinheit 12 einlegbar ist, mittels welcher letztlich die Rotorwelle 32, und damit das Förderelement, in Rotation versetzbar ist.

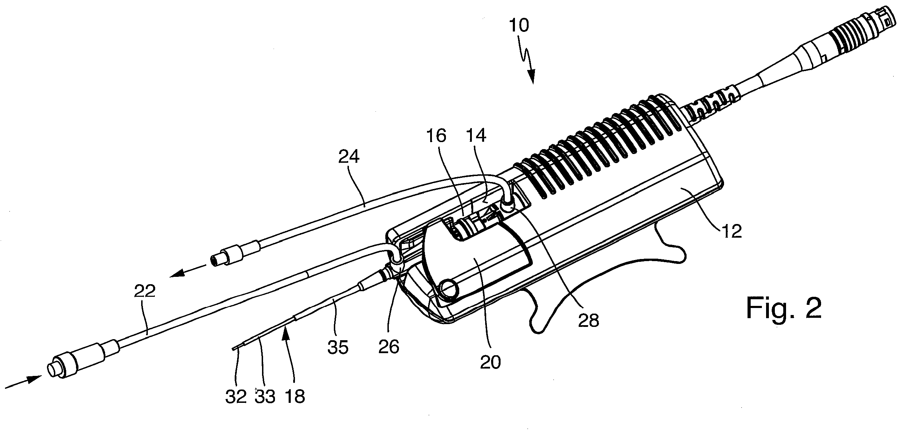

In der Figur 2 ist eine Antriebseinheit 12 einer erfindungsgemäßen Katheterpumpe 10 gezeigt. Die Antriebseinheit 12 weist eine Aufnahme 14 auf. Ferner ist das proximale Ende 16 eines Katheters 18 gezeigt, der an seinem distalen, nicht dargestellten Ende das im Betrieb rotierende Förderelement, aufweist.

Das proximale Ende 16 des Katheters 18 ist dabei in der Aufnahme 14 angeordnet und wird dort mittels eines Halteelements 20 sicher gehalten.

Am proximalen Ende 16 sind zwei Schläuche 22, 24 vorgesehen. Über den Schlauch 22 kann über einen Einlauf 26 Spül- und Schmierflüssigkeit in den Katheter 18 eingeleitet werden. Diese Spül- und Schmierflüssigkeit wird durch den Katheter 18 zum Pumpenkopf 15 geleitet. Im Pumpenkopf 15 wird ein Teil dieser Schmier- und Spülflüssigkeit wieder durch den Katheter 18 zurückgeführt und über einen Auslauf 28 und den Schlauch 24 ausgelassen. Die rückgeführte Schmier- und Spülflüssigkeit wird dabei zwischen der sich im Betrieb drehenden Rotorwelle 32 und einem Innenkatheter 33 rückgeführt. Der Innenkatheter 33 wird dabei von einem Außenkatheter 35 umgeben, wobei Schmier-und Spülflüssigkeit über das Lumen zwischen dem Innenkatheter 33 und dem Außenkatheter 35 hin zum Pumpenkopf 15 gefördert wird.

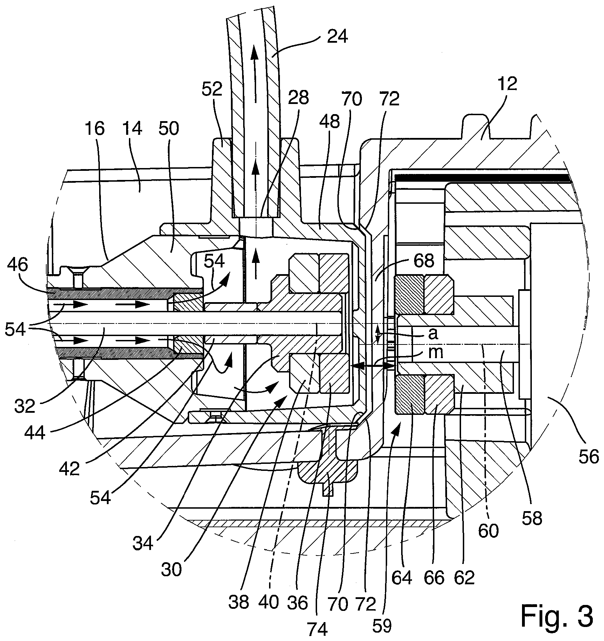

In dem in der Figur 3 gezeigten Ausschnitt ist das proximale Ende 16 mit dem Auslauf 28 in der Aufnahme 14 gezeigt.

Deutlich zu erkennen ist, dass das proximale Ende 16 des Katheters 18 einen Koppelabschnitt 30 aufweist, der drehfest mit der Rotorwelle 32 verbunden ist. Dazu sieht der Koppelabschnitt 30 einen Magnethalter 34 vor, auf dem zum einen ein Magnetring 36 mit Magnetelementen und zum anderen ein Weicheisenring 38 zur entsprechenden Beeinflussung des Magnetfelds angeordnet sind. Der Koppelabschnitt 30 bzw. der Magnetring 36 ist dabei im Betrieb um die Koppelachse 40, die in der Rotorwellenachse liegt, verdrehbar. Die Rotorwelle 32 als solche ist über einen Schaft 42 und über ein Lager 44 in einer Lagerhülse 46 am proximalen Ende 16 des Katheters 18 drehbar gelagert.

Wie aus Figur 3 ferner deutlich wird, ist am freien Ende des Katheters eine den Koppelabschnitt 30 umgebende Kappe 48 vorgesehen, welche fluiddicht an einem die Hülse 46 aufnehmenden Hülsengehäuse 50 angeordnet ist. Die Kappe 48 sieht dabei den Auslauf 28 und einen Aufnahmestutzen 52 für den Schlauch 24 vor. Insgesamt kann im Betrieb der Katheterpumpe 10 die Schmier- und Spülflüssigkeit zwischen der Lagerhülse 46 und der rotierenden Rotorwelle 32 in Axialrichtung, wie mit den Pfeilen 54 angedeutet, das Lager 44 hin zum Koppelabschnitt 32 durchströmen und durch den Auslauf 28 in den Schlauch 24 abfließen.

In der Antriebseinheit 12 ist, wie ebenfalls in Figur 3 deutlich wird, ein Antrieb 56 in Form eines Elektromotors vorgesehen. Der Antrieb 56 kann dabei eine geeignete Getriebeeinheit umfassen. Der Antrieb 56 treibt einen Antriebsabschnitt 59 an, der eine um eine Antriebsachse 60 antreibbare Antriebswelle 58 und einen Magnethalter 62 vorsieht, auf welchem ein Magnetring 64 und ein Weicheisenring 66 angeordnet sind.

Die Magnetringe 36 und 64 sind dabei komplementär so zueinander ausgebildet, dass bei einer rotatorischen Drehung des Antriebsabschnitts 59 der Koppelabschnitt 30 zur gegenseitigen Drehkopplung in Rotation versetzt wird. Der Durchmesser des Magnetrings 64 entspricht dabei vorzugsweise dem Durchmesser des Magnetrings 36.

Wie zudem aus Figur 3 deutlich wird, sind die Koppelachse 40 und die Antriebsachse 60 in Querrichtung um einen Abstand a voneinander beabstandet bzw. versetzt zueinander angeordnet. Der Abstand a ist dabei so gewählt, dass das in den Ringmagneten 36 und 64 erzeugte Magnetfeld eine Kraft bereitstellt, die den Koppelabschnitt 36 in eine quer zur Koppelachse 40 verlaufende Richtung drängt.Durch Bereitstellen der quer zur Kopplungsachse verlaufenden Kraft kann auch bei höheren Drehzahlen der Rotorwelle ein vergleichsweise geräuscharmer Betrieb der Katheterpumpe bereitgestellt werden.

Der axiale Abstand m der Magnetringe 36 und 64 zueinander liegt vorzugsweise im Bereich von 3 mm bis 4 mm. Der Abstand a der beiden Achsen 40 und 60 liegt vorzugsweise im Bereich von 1,5 mm bis 2,5 mm. Der Abstand a ist dabei geringfügig kleiner als der Durchmesser der Rotorwelle 32. Die Abstände a und m können allerdings erfindungsgemäß auch variieren und sind Abhängig von der Magnetkraft, der Ausführung und der Anzahl der Magnetelemente bzw. dem zu übertragenden Drehmoment.

Um zu verhindern, dass Flüssigkeit oder Medien in den Antriebsabschnitt 59 oder den Antrieb 56 eindringen können, ist zwischen der Aufnahme 14 und dem Antriebsabschnitt 59 eine fluiddichte Wandung 68 vorgesehen.

Um ein positionsgenaues Einlegen des proximalen Endes 16 des Katheters 18 in die Aufnahme 14 zu gewährleisten, weist die Kappe 48 an der dem Antriebsabschnitt 59 zugewandten Seiten Zentrierschrägen 70 auf, die mit an der Wandung 68 vorgesehenen Zentrierschrägen 72 korrespondieren.

Um mögliche Restschwingungen zu dämpfen, bzw. um diesen keinen Resonanzkörper zu geben, weist die Aufnahme 14 bzw. eine die Aufnahme 14 begrenzende Wandung ein oder mehrere Dämpfungselemente 74 auf, die gegen den Koppelabschnitt 30 bzw. dessen Kappe 48 wirken.

Die Magnetelemente der Magnetringe 36 und 64 sind dabei derart ausgebildet, dass eine Drehkopplung durch den Wandungsabschnitt 68 und durch den dem Wandungsabschnitt zugewandten Abschnitt der Kappe 48 möglich ist.

Bei der Auslegung der Drehkoppelung kann die Eigenschaft einer Entkoppelung bei zu hohem Drehmoment oder zu hoher Drehzahl genutzt werden. Mit steigender Belastung verdrehen sich die beiden korrespondierenden Magnetringe 36 und 64 mehr und mehr gegeneinander, wobei ab einem Grenzdrehmoment bzw. bei Überschreiten einer Grenzdrehzahl der Verdrehwinkel zu groß wird, und damit eine Entkoppelung stattfinden. Diese Eigenschaft kann als Überlastschutz Verwendung finden.

## Patentansprüche

1. Katheterpumpe (10) mit einer Antriebseinheit (12) und einem Katheter (18), wobei der Katheter (18) einen Pumpenkopf (15) zum Einführen in das arterielle Gefäßsystem, eine verdrehbar angeordnete Rotorwelle (32) zum Antrieben eines am Pumpenkopf (10) vorgesehenen, expandierbaren Förderelements und an seinem proximalen Ende (16) einen mit der Rotorwelle (32) drehfest verbundenen, um eine Koppelachse (40) rotierbaren Koppelabschnitt (30) aufweist, und wobei die Antriebseinheit (12) einen Antrieb (56) und einen vom Antrieb (56) um eine Antriebsachse (60) rotierend antreibbaren Antriebsabschnitt (59) aufweist,
wobei der Koppelabschnitt (30) und/oder der Antriebsabschnitt (59) Magnetelemente zur berührungslosen gegenseitigen Drehkopplung aufweisen,
**dadurch gekennzeichnet,**
**dass** die Koppelachse (40) und die Antriebsachse (56) in Querrichtung um einen Abstand a, der im Bereich von 0,7 mm bis 6,5 mm und vorzugsweise im Bereich von 1 mm bis 3 mm und weiter vorzugsweise im Bereich von 2 mm liegt, derart zueinander beabstandet sind, dass das Magnetfeld der Magnetelemente den Koppelabschnitt (30) in eine quer zur Koppelachse (40) verlaufende Richtung drängt.

2. Katheterpumpe (10) nach Anspruch 1, **dadurch gekennzeichnet, dass** der Koppelabschnitt (30) zum Antriebsabschnitt (59) in axialer Richtung einen Abstand m aufweist, wobei der Abstand m im Bereich von 2 mm bis 6 mm und vorzugsweise im Bereich von 3 mm bis 5 mm und weiter vorzugsweise im Bereich von 3,5 mm liegt.

3. Katheterpumpe (10) nach einem der Ansprüche 1 oder 2, **dadurch gekennzeichnet, dass** die Rotorwelle (32) einen Durchmesser d aufweist, wobei der Abstand a im Bereich des 0,5 bis 1,5 fachen, und vorzugsweise im Bereich des 0,8 bis 1,2 fachen, des Durchmessers d liegt.

4. Katheterpumpe (10) nach Anspruch 3, **dadurch gekennzeichnet, dass** der Durchmesser d im Bereich von 0,7 mm bis 4,5 mm und vorzugsweise im Bereich von 1,5 mm bis 3 mm und weiter vorzugsweise im Bereich von 2 mm liegt.

5. Katheterpumpe (10) nach einem der vorhergehenden Ansprüche, **dadurch gekennzeichnet, dass** die Antriebseinheit (12) eine Aufnahme (14) für den Koppelabschnitt (30) aufweist, wobei der Koppelabschnitt (30) in der Aufnahme (14) eine bezüglich des Antriebsabschnitts (59) in Querrichtung um den Abstand a versetzte Betriebslage einnimmt.

6. Katheterpumpe (10) nach Anspruch 5, **dadurch gekennzeichnet, dass** die Aufnahme (14) oder eine die Aufnahme (14) begrenzende Wandung ein oder mehrere Dämpfungselemente (74) vorsieht, die zur Schwingungsdämpfung gegen den Koppelabschnitt (30) wirken.

7. Katheterpumpe (10) nach einem der vorhergehenden Ansprüche, **dadurch gekennzeichnet, dass** der Koppelabschnitt (30) einen an dem freien Ende der Rotorwelle (32) angeordneten Magnethalter (34) mit daran angeordneten Magnetelementen (36) aufweist.

8. Katheterpumpe (10) nach einem der vorhergehenden Ansprüche, **dadurch gekennzeichnet, dass** der Koppelabschnitt (30) von einer fluiddichten Kappe (48) umgeben ist.

9. Katheterpumpe (10) nach Anspruch 8, **dadurch gekennzeichnet, dass** die Kappe (48) an der dem Antriebsabschnitt (59) zugewandten Seite und/oder die Aufnahme (14) an der dem Koppelabschnitt (30) zugewandten Seite Zentrierschrägen (70) zum Zentrieren der Kappe (48) in der Betriebslage aufweist.

10. Katheterpumpe (10) nach Anspruch 8 oder 9, **dadurch gekennzeichnet, dass** die Kappe (48) einen Auslauf (28) aufweist und dass der Katheter (18) derart ausgebildet ist, dass die Rotorwelle (32) im Betrieb von einer Spülflüssigkeit umspült wird, wobei die Spülflüssigkeit über den Auslauf (28) abgeführt wird.

11. Katheterpumpe (10) nach einem der vorhergehenden Ansprüche, **dadurch gekennzeichnet, dass** die Antriebseinheit (12) zwischen der Aufnahme (14) und dem Antriebabschnitt (59) einen fluiddichten Wandungsabschnitt (68) aufweist.

12. Katheterpumpe (10) nach einem der vorhergehenden Ansprüche, **dadurch gekennzeichnet, dass** die Magnetelemente am Koppelabschnitt (30) und/oder am Antriebsabschnitt (59) zur Drehkoppelung so ausgelegt sind, dass bei einem Grenzdrehmoment die Magnetkoppelung abreist.

## Claims

1. Catheter pump (10) with a drive unit (12) and a catheter (18), wherein the catheter (18) comprises a pump head (15) for insertion into the arterial vascular system, a rotor shaft (32) arranged rotatably for driving an expandable conveyor element provided at the pump head (10) and, at its proximal end (16), a coupling section (30) connected in a rotatably fixed manner to the rotor shaft (32) and rotatable around a coupling axis (40), and wherein the drive unit (12) comprises a drive (56) and a drive section (59) driveable by the drive (56) around the drive axis (60) in a rotating manner, wherein the coupling section (30) and/or the drive section (59) comprise magnetic elements for contact-free mutual rotational coupling,
**characterized in that**
the coupling axis (40) and the drive axis (56) are set apart in a transverse direction by a distance a which is in a range from 0.7 to 6.5 mm and preferably in a range from 1 mm to 3 mm and further preferably in a range of 2 mm, in such a way that the magnetic field of the magnetic elements urges the coupling section (30) in a direction extending transverse to the coupling axis (40).

2. Catheter pump (10) as claimed in claim 1, **characterized in that** the coupling section (30) has a distance m to the drive section (59) in an axial direction, wherein the distance m is in a range from 2 mm to 6 mm and preferably in a range from 3 mm to 5 mm and further preferably in a range of 3.5 mm.

3. Catheter pump (10) as claimed in one of claims 1 or 2, **characterized in that** the rotor shaft (32) has a diameter d, wherein the distance a is in a range of 0.5- to 1.5-fold and preferably 0.8- to 1.2-fold of the diameter d.

4. Catheter pump (10) as claimed in claim 3, **characterized in that** the diameter d is in a range from 0.7 mm to 4.5 mm and preferably in a range from 1.5 mm to 3 mm and further preferably in a range of 2 mm.

5. Catheter pump (10) as claimed in one of the preceding claims, **characterized in that** the drive unit (12) comprises a seat (14) for the coupling section (30), wherein the coupling section (30) takes an operating position in the seat (14) which is offset with respect to the drive section (59) in a transverse direction by the distance a.

6. Catheter pump (10) as claimed in claim 5, **characterized in that** the seat (14) or a wall delimiting the seat (14) provides one or more damping elements (74) effective for damping oscillations with respect to the coupling section (30).

7. Catheter pump (10) as claimed in one of the preceding claims, **characterized in that** the coupling section (30) comprises a magnet support (34) arranged at a free end of the rotor shaft (32) with magnetic elements (36) arranged theron.

8. Catheter pump (10) as claimed in one of the preceding claims, **characterized in that** the coupling section (30) is surrounded by a fluid-tight cap (48).

9. Catheter pump (10) as claimed in claim 8, **characterized in that** the cap (48), on the side facing the drive section (59), and/or the seat (14), on the side facing the coupling section (30), comprise centering bevels (70) for centering the cap (48) in its operating position.

10. Catheter pump (10) as claimed claim 8 or 9, **characterized in that** the cap (48) comprises a discharge opening (28) and that the catheter (18) is configured in such a way that the rotor shaft (32) is flushed by a flushing liquid during operation, wherein the flushing liquid is discharged through the discharge opening (28).

11. Catheter pump (10) as claimed in one of the preceding claims, **characterized in that** the drive unit (12) has a fluid-tight wall section (68) between the seat (14) and the drive section (59).

12. Catheter pump (10) as claimed in one of the preceding claims, **characterized in that** the magnetic elements on the coupling section (30) and/or on the drive section (59) are configured in such a way for rotational coupling that the magnetic coupling is separated at a torque limit.

## Revendications

1. Pompe à cathéter (10) avec une unité d'entraînement (12) et un cathéter (18), dans laquelle le cathéter (18) présente une tête de pompe (15) à introduire dans le système vasculaire artériel, un arbre de rotor (32) disposé en rotation, destiné à entraîner un élément de refoulement expansible, prévu sur la tête de pompe (15), et à son extrémité proximale (16) une partie d'accouplement (30) reliée de manière solidaire en rotation à l'arbre de rotor (32), pouvant tourner autour d'un axe d'accouplement (40), et dans laquelle l'unité d'entraînement (12) présente un entraînement (56) et une partie d'entraînement (59) pouvant être entraînée en rotation autour d'un axe d'entraînement (60) par l'entraînement (56), dans laquelle la partie d'accouplement (30) et/ou la partie d'entraînement (59) présentent des éléments magnétiques pour l'accouplement mutuel en rotation sans contact,
**caractérisée en ce**
**que** l'axe d'accouplement (40) et l'axe d'entraînement (56) sont espacés l'un de l'autre dans la direction transversale d'une distance a, qui se situe dans la plage de 0,7 mm à 6,5 mm et de préférence dans la plage de 1 mm à 3 mm et mieux encore dans la plage de 2 mm, de telle sorte que le champ magnétique des éléments magnétiques pousse la partie d'accouplement (30) dans une direction s'étendant transversalement à l'axe d'accouplement (40).

2. Pompe à cathéter (10) selon la revendication 1, **caractérisée en ce que** la partie d'accouplement (30) présente par rapport à la partie d'entraînement (59) dans la direction axiale une distance m, dans laquelle la distance m se situe dans la plage de 2 mm à 6 mm et de préférence dans la plage de 3 mm à 5 mm et mieux encore dans la plage de 3,5 mm.

3. Pompe à cathéter (10) selon l'une quelconque des revendications 1 ou 2, **caractérisée en ce que** l'arbre de rotor (32) présente un diamètre d, dans laquelle la distance a se situe dans la plage de 0,5 à 1,5 fois, et de préférence dans la plage de 0,8 à 1,2 fois le diamètre d.

4. Pompe à cathéter (10) selon la revendication 3, **caractérisée en ce que** le diamètre d se situe dans la plage de 0,7 mm à 4,5 mm et de préférence dans la plage de 1,5 mm à 3 mm et mieux encore dans la plage de 2 mm.

5. Pompe à cathéter (10) selon l'une quelconque des revendications précédentes, **caractérisée en ce que** l'unité d'entraînement (12) présente un logement (14) pour la partie d'accouplement (30), dans laquelle la partie d'accouplement (30) occupe dans le logement (14) une position de fonctionnement décalée de la distance a dans la direction transversale par rapport à la partie d'entraînement (59).

6. Pompe à cathéter (10) selon la revendication 5, **caractérisée en ce que** le logement (14) ou une paroi délimitant le logement (14) prévoit un ou plusieurs éléments d'amortissement (74), qui agissent contre la partie d'accouplement (30) pour amortir les vibrations.

7. Pompe à cathéter (10) selon l'une quelconque des revendications précédentes, **caractérisée en ce que** la partie d'accouplement (30) présente un support magnétique (34), disposé à l'extrémité libre de l'arbre de rotor (32), avec des éléments magnétiques (36) disposés dessus.

8. Pompe à cathéter (10) selon l'une quelconque des revendications précédentes, **caractérisée en ce que** la partie d'accouplement (30) est entourée par un capuchon (48) étanche au fluide.

9. Pompe à cathéter (10) selon la revendication 8, **caractérisée en ce que** le capuchon (48) présente sur la face tournée vers la partie d'entraînement (59) et/ou le logement (14), sur la face tournée vers la partie d'accouplement (30), des chanfreins de centrage (70) permettant le centrage du capuchon (48) dans la position de fonctionnement.

10. Pompe à cathéter (10) selon la revendication 8 ou 9, **caractérisée en ce que** le capuchon (48) présente une évacuation (28) et que le cathéter (18) est réalisé de telle sorte que l'arbre de rotor (32), pendant le fonctionnement, est baigné par un liquide de lavage, dans laquelle le liquide de lavage est évacué par l'intermédiaire de l'évacuation (28).

11. Pompe à cathéter (10) selon l'une quelconque des revendications précédentes, **caractérisée en ce que** l'unité d'entraînement (12) présente entre le logement (14) et la partie d'entraînement (59) une partie de paroi (68) étanche au fluide.

12. Pompe à cathéter (10) selon l'une quelconque des revendications précédentes, **caractérisée en ce que** les éléments magnétiques sur la partie d'accouplement (30) et/ou sur la partie d'entraînement (59) sont conçus pour un accouplement en rotation, de sorte que, lors d'un couple limite, l'accouplement magnétique se défait.
